(19) Europäisches Patentamt
European Patent Office
Office européen des brevets

(11) Veröffentlichungsnummer: 0 352 529
A2

(12) **EUROPÄISCHE PATENTANMELDUNG**

(21) Anmeldenummer: 89112509.8

(22) Anmeldetag: 08.07.89

(51) Int. Cl.⁴: A01N 43/40, A01N 43/54, A01N 43/58, A01N 43/60

(30) Priorität: 23.07.88 DE 3825172

(43) Veröffentlichungstag der Anmeldung: 31.01.90 Patentblatt 90/05

(84) Benannte Vertragsstaaten: AT BE CH DE ES FR GB IT LI NL

(71) Anmelder: BAYER AG
D-5090 Leverkusen 1 Bayerwerk(DE)

(72) Erfinder: Alig, Bernd, Dr.
Gartenstrasse 2
D-5330 Königswinter 21(DE)
Erfinder: Stendel, Wilhelm, Dr.
In den Birken 55
D-5600 Wuppertal 1(DE)
Erfinder: Londershausen, Michael, Dr.
Galileistrasse 11
D-4006 Erkrath 2(DE)

(54) **Mittel zur Bekämpfung von Flöhen.**

(57) Die vorliegende Erfindung betrifft die Verwendung von substituierten Alkoxydiphenylethern der allgemeinen Formel

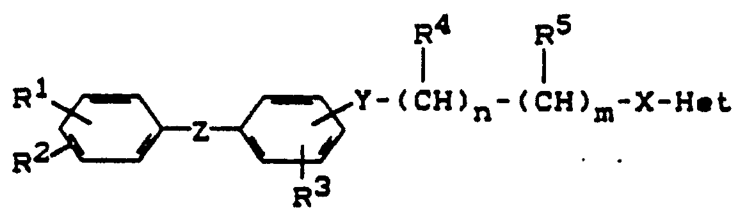

in welcher
$R^1$ für Wasserstoff, Halogen, Alkyl, Alkoxy, Alkylthio, Halogenalkyl, Halogenalkoxy, Halogenalkylthio, Dioxyalkylen, Dioxyhalogenalkylen, CN, $NO_2$, Alkenyl, Alkinyl, Alkoxyalkyl, Alkoxyalkoxy, Hydroxyalkoxy steht,
$R^2$ für die bei $R^1$ angegebenen Reste steht,
$R^3$ für die bei $R^1$ angegebenen Reste steht,
$R^4$ für Wasserstoff, Alkyl, Halogenalkyl oder Halogen steht,
$R^5$ für die bei $R^4$ angegebenen Reste steht,
Het für gegebenenfalls substituiertes Heteroaryl steht das nicht über das Heteroatom an den übrigen Rest gebunden ist,
X, Y, Z unabhängig voneinander für O, S oder $CH_2$ steht,
m und n unabhängig voneinander für 0, 1, 2, 3 stehen ihre Summe aber ≥ 2 ist,
zur Bekämpfung von Flöhen sowie dafür geeignete Mittel.

EP 0 352 529 A2

## Mittel zur Bekämpfung von Flöhen

Die vorliegende Erfindung betrifft die Verwendung von substituierten Alkoxydiphenylethern und -diphenylmethanen zur Bekämpfung von Flöhen bei Haustieren.

Alkoxydiphenylether und -diphenylmethane und ihre insektizide Wirkung sind bereits bekannt (EP-OS 128 648). Es ist jedoch nichts über ihre Wirkung gegen Flöhe bekannt geworden.

Erwachsene Flöhe leben normalerweise im Haarkleid der Wirtstiere. Sie ernähren sich von deren Blut und legen ihre Eier im Haarkleid des Wirtstiers ab.

Da die abgelegten Eier nicht am Haarkleid haften, fallen sie rasch ab und können in der gesamten Umgebung der Wirtstiere z.B. Lager und Liegestätten gefunden werden. Das bedeutet, daß der gesamte Lebensraum der Wirtstiere mit Floheiern verseucht ist, aus denen innerhalb von wenigen Tagen Flohlarven schlüpfen. Man unterscheidet bei den Larven drei Entwicklungsstadien, die jeweils ca. drei Tage dauern. Das 3. Larvenstadium spinnt einen Kokon und verpuppt sich. Unter günstigen Bedingungen (20-30 °C, 60-70 % relativer Luftfeuchtigkeit) dauert die Entwicklung vom Ei zur Puppe ca. 10 Tage. Nach etwa weiteren 8 Tagen ist die Entwicklung der Flöhe abgeschlossen und in den auf dem Boden, Teppichen, Schlafplätzen usw. liegenden Kokons finden sich schlüpfbereite Flöhe. Der Jungfloh kann monatelang in seinem Kokon bleiben. Unter ungünstigen Bedingungen kann die Entwicklung vom Ei bis zum erwachsenen Jungfloh jedoch auch 4 bis 5 Monate dauern. Flöhe benötigen zur Erlangung ihrer Geschlechtsreife, d.h. zur Ablage fertiger Eier Blut als Nahrungsmittel. Dieses Blut stammt idealerweise vom jeweils spezifischen Wirt. Flöhe können aber auch, besonders nach längerem Fasten das Blut anderer Wirte akzeptieren.

Der Flohbefall der Haustiere wie Hunde und Katzen ist für das befallene Tier nicht nur lästig, sondern er fügt den befallenen Tieren auch erhebliche Schmerzen zu. Flöhe können außerdem verschiedene Bandwurmarten übertragen. Sie stellen daher auch ein medizinisches Problem für die befallenen Tiere sowie die Tierhalter dar. Auch der Tierhalter kann von Flöhen gebissen werden. Bei manchen Menschen führt dies zu Flohstichallergie. Eine wirksame Bekämpfung der Flöhe bei Hunden und Katzen war deshalb schon von jeher wünschenswert und notwendig, zumal diese Haustiere in einen zunehmend engeren Kontakt mit dem Menschen leben.

Die Bekämpfung von Flöhen bei Haustieren die mit Menschen zusammen leben, wie z.B. Hunde und Katzen, erfolgt

1. durch Behandlung des Wirtstiers,
2. durch Behandlung der Liegestellen des Wirtstiers,
3. durch Behandlung der gesamten Umgebung des Wirtstiers d.h. der Wohnung und der Bereiche außerhalb der Wohnung des Menschens.

Besondere Bedeutung für eine gründliche Flohbekämpfung hat die Behandlung der Liegestellen und der Umgebung des Wirtstieres. Eine Behandlung des Wirtstieres allein ist wenig erfolgversprechend, da über Wochen sogar Monate die Gefahr des Wiederbefalls durch neu ausgeschlüpfte Jungflöhe aus der Umgebung gegeben ist. Besonders erfolgversprechend ist die Behandlung von Liegestellen und Umgebung dann, wenn nicht nur die adulten Flöhe sondern auch die Entwicklungsstadien, die getrennt vom Wirtstier ablaufen, beeinflußt werden können.

Es ist bekannt, daß die Behandlung mit Insektiziden wie z.B. Phosphorsäureestern, Carbamaten, natürlichen und synthetischen Pyrethroiden durchgeführt werden kann. Die dabei eingesetzten Wirkstoffe müssen neben geringer Säugetiertoxizität auch eine lang anhaltende Wirkung besitzen um allzu häufige Behandlungen zu vermeiden. Die Wirkstoffe müssen außerdem sowohl gegen adulte als auch larvale Stadien des Flohs wirksam sein. Die oben angeführten Wirkstoffe sind z.T. nur spezifisch gegen adulte Flöhe wirksam, z.T. besitzen sie eine zu geringe Stabilität und damit verbunden eine zu kurze Wirkungsdauer, z.T. sind sie toxisch und können nur unter Beachtung besonderer Vorsichtsmaßnahmen angewendet werden.

Es ist auch bekannt, daß Insektenentwicklungshemmer wie Methoprene zur Flohbekämpfung eingesetzt werden können. Methoprene ist jedoch instabil und muß daher, um über längere Zeit wirksam zu sein, in relativ hohen Konzentrationen angewendet werden. Andererseits wirkt Methoprene nicht gegen adulte Flöhe. Es kann somit leicht zu erneutem Flohbefall kommen, außerdem wird die direkte Belästigung von Mensch und Tier durch die adulten Flöhe nicht beseitigt.

Durch diese Mängel der bekannten Insektizide und Entwicklungshemmer bestand Bedürfnis nach Wirkstoffen gegen Flöhe oder einzelne Entwicklungsstadien der Flöhe die hochwirksam, stabil und von geringer Säugetiertoxizität sind.

Es wurde nun gefunden, daß sich die substituierten Alkoxydiphenylether oder -diphenylmethane der allgemeinen Formel I

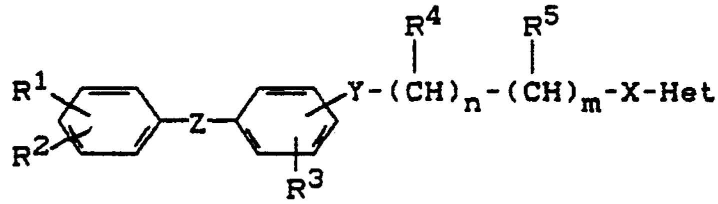 (I)

in welcher
$R^1$ für Wasserstoff, Halogen, Alkyl, Alkoxy, Alkylthio, Halogenalkyl, Halogenalkoxy, Halogenalkylthio, Dioxyalkylen, Dioxyhalogenalkylen, CN, $NO_2$, Alkenyl, Alkinyl, Alkoxyalkyl, Alkoxyalkoxy, Hydroxyalkoxy steht,
$R^2$ für die bei $R^1$ angegebenen Reste steht,
$R^3$ für die bei $R^1$ angegebenen Reste steht,
$R^4$ für Wasserstoff, Alkyl Halogenalkyl oder Halogen steht,
$R^5$ für die bei $R^4$ angegebenen Reste steht,
Het für gegebenenfalls substituiertes Heteroaryl steht das nicht über das Heteroatom an den übrigen Rest gebunden ist,
X, Y unabhängig voneinander für -O-, -S- stehen
Z für -O-, -S-, $-CH_2-$, $-CHCH_3-$, $-C(CH_3)_2-$ steht,
m und n unabhängig voneinander für 0, 1, 2, 3 stehen ihre Summe aber gleich oder größer 2 ist,
hervorragend zur Bekämpfung von Flöhen eignen.

Wie bereits erwähnt ist aus EP-OS 128 648 bekannt, daß Verbindungen der Formel I als Insektenentwicklungshemmer insektizid wirksam sind. Dies wird auch anhand verschiedener Schadinsekten wie z.B. Galleria mellonella, Culex pipiens pallens, Musca domestica und Spinnmilben z.B. Tetranychus cinnabarinus gezeigt.

Allerdings geht aus dieser Literaturstelle nichts über die hervorragende Eignung dieser Verbindungen zur Bekämpfung von Flöhen hervor. Die Wirkung der Verbindungen wird als juvenilhormon-ähnlich charakterisiert. Gerade für Verbindungen mit solchen Wirkungen ist es äußerst schwierig klare Vorhersagen darüber zu treffen ob sie gegen eine Ordnung von Insekten wirksam sind die sich in ihrer Lebensweise und in ihrem Entwicklungsgang deutlich von den beschriebenen Insekten unterscheidet.

Eine klare Vorhersage der insektiziden Wirksamkeit über alle Ordnungen und Entwicklungsstadien von Insekten hinweg ist, selbst wenn diese Wirksamkeit nur an wenigen Spezies belegt ist, z.B. dann möglich wenn die Wirksamkeit auf einem Mechanismus beruht der allen Ordnungen sowie Entwicklungsstadien gemeinsam ist. Dazu gehört z.B. die Hemmung der Acetylcholinesterase durch Phosphorsäureester, Carbamate und Pyrethroide. Diese Verbindungen wirken z.B. neurotoxisch.

Untersucht man die maximalen Aktivitätsunterschiede die innerhalb einer Substanzklasse für einzelne Insektenordnungen und Arten charakteristisch sind, dann findet man bei neurotoxisch klassifizierten Insektiziden nur eine geringe Variation, verglichen mit den als Entwick lungshemmer einzuordnenden Insektiziden. D.h. die Unterschiede zwischen den wirksamen Konzentrationen einzelner Wirkstoffe für einzelne Ordnungen von Insekten sind vergleichsweise gering.

Anders dagegen bei Wirkstoffen die die Insektenentwicklung beeinflussen wie z.B. bei juvenilhormonanalogen Verbindungen. Bei diesen Verbindungen bestehen große Unterschiede zwischen den wirksamen Konzentrationen einzelner Wirkstoffe für verschiedene Insektenordnungen.

Um solche Unterschiede aus den Angaben der Literatur vergleichen zu können, wurden Variationsfaktoren aus den aufgebrachten Substanzmengen pro µg Tier-Frischgewicht bzw. pro µg Tier dadurch errechnet, daß man für jeden Wirkstoff die maximal notwendige Aufwandmenge durch die minimale notwendige Aufwandmenge dividierte. Dieser Faktor ist ein Maß für die unterschiedlichen Aufwandmengen, die für einen Wirkstoff bei verschiedenen Insektenordnungen erforderlich sind.

Ein Vergleich von neurotoxischen (212 Substanzen) und juvenoid/entwiclungsinhibitorisch (330 Substanzen) wirksamen Verbindungen gegen verschiedene Insektenordnungen ergab dabei mittlere Variationsfaktoren von ca. 51 für die neurotoxischen Insektizide und 2187 für die Entwicklungsinhibitoren, zu denen auch die Juvenilhormonanalogen gehören.

Die der Errechnung dieser Variationsfaktoren zugrunde liegenden Werte wurden folgenden Literaturstellen entnommen:

1) C.A.Henrick, 1982
JUVENILE HORMONE ANALOGS: STRUCTURE-ACTIVITY RELATIONSHIPS In: Insecticide Mode of Action, 11 (314-402), Ed: J.R. Coats, Academic Press

2) G.S.F. Ruigt, 1985

PYRETHROIDS
In: Comprehensive Insect Physiology Biochemistry and Pharmacology, 12 (184-262), Eds: G.A. Kerkut and G.I. Gilbert, Pergamon Press

3) C.O. Knowles, 1982
STRUCTURE-ACTIVITY RELATIONSHIPS AMONG AMIDINE ACARICIDES AND INSECTICIDES
In: Insecticide Mode of Action, 9 (243-277), Ed: J.R. Coats, Academic Press

4) T.A. Magee, 1982
OXIME-CARBAMATE INSECTICIDES
In: Insecticide Mode of Action, 4 (71-100), Ed: J.R. Coats Academic Press

5) H. Ohkawa, 1982
STEREOSELECTIVITY OF ORGANOPHOSPHORUS INSECTICIDES
In: Insecticide Mode of Action, 6 (163-185), Ed: J.R. Coats, Academic Press

6) A. Retnakaran and J.E. Wright, 1987
CONTROL OF INSECT PESTS WITH BENZOYLPHENYL-UREAS
In: Chitin and Benzoylphenyl-Ureas, Eds: J.D. Wright and A. Retnakran, Dr. J. Junk Pubilshers

Die $LC_{50}$ bzw. $LC_{95}$-Werte sind in den verschiedenen Literaturstellen und durch die Testkonstruktionen bedingt auf ppm-Werte (im Zuchtmedium) bezogen, oder es werden die aufgebrachten Substanzmengen pro Tier bzw. Tier-Frischgewicht angegeben, was dann in der Aktivitätsbestimmung als µg/Tier bzw. µg/Tier-Frischgewicht ausgewertet wird.

Uneinheitliche Dimensionen dieser Art finden sich im Aktivitätsvergleich der Juvenilhormonmimics (Aedes aegypti und Heliothis virescens Werte sind in ppm angegeben, Galleria-, Tenebrio- und Musca-Werte dagegen in µg pro Tier: Lit. 1). Da die Variationsfaktoren beider Bestimmungstypen nicht zu vergleichen sind, wurden die ppm-Werte getrennt von den µg/Tier-Werten ausgewertet, und weiterhin die µg/Tier-Werte auf µg/Tier-Frischgewicht bezogen, um die völlig unterschiedlichen Tiergrößen/gewichte auf eine gemeinsame Basis zu beziehen. Dabei ergaben sich folgende Faktoren, mit denen die Meßwerte multipliziert wurden, um eine Basis zu besitzen:

Tabelle

| Art | Mittelwert g-Frischgewicht | n | Faktor |
|---|---|---|---|
| 1 Galleria mellonella | 0,400 g/Puppe | 5 | 2,50 |
| 2 Tenebrio molitor | 0,61 g/Puppe | 12 | 6,25 |
| 3 Musca domestica | 0,034 g/Vorpuppe | 100 | 29,41 |

Nicht in die Auswertung einbezogen wurden Werte, bei denen die µg/Tier-Werte für die drei Arten alle über > 100 Faktor lagen (ca. 11 % aller getesteten JH-Mimics), und generell die Bestimmungen, bei denen Einzelwerte fehlten.

In einzelnen ergaben sich folgende Werte:

| Substanzklassen | Variationsfaktor (x) | Standardabweichung/Mittelwert $S_{n-1/x}$ | Zahl der Wirkstoffe n |
|---|---|---|---|
| Neurotische Insektizide: | | | |
| Organophosphate | 125 | 1,43 | 12 |
| Carbamate | 45 | 1,82 | 130 |
| Pyrethroide | 4,4 | 2,47 | 51 |
| Amidine | 29 | 0,80 | 19 |
| Entwicklungsinhibitoren: | | | |
| Benzoylphenyl-Harnstoffe | 2970 | 1,99 | 6 |
| Juvenilhormon-Mimics (µg/g-Frischgewicht-Werte) | 2727 | 3,11 | 177 |
| Juvenilhormon-Mimics (ppm-Werte) | 865 | 1,80 | 147 |

Die Werte zeigen, daß juvenilhormon-wirksame Verbindungen eine wesentlich größere Variation zwischen wirksamen und unwirksamen Konzentrationen aufweisen als dies bei neurotoxisch wirksamen Verbindungen der Fall ist.

Diese Aussage bedeutet, daß man im Bereich der neurotoxischen Insektizide, auch bei unterschiedlichen Dosis/Empfindlichkeitsbeziehungen verschiedener Insektenordnungen und Arten, im Mittel die Wirkung auf eine nicht getestete Insektenart besser vorhersagen kann, als dies bei Entwicklungsinhibitoren möglich ist.

Der Vergleich der mittleren Variationsfaktoren neurotoxischer Insektizide (x = 50,85) und Entwicklungsinhibitoren (x = 2187) zeigt, daß man die Wirkung neurotoxischer Insektizide etwa um den Faktor 43 (2187:50,85) genauer vorhersagen kann, als dies bei Entwicklungsinhibitoren der Fall ist.

Dies läßt sich auch an folgendem Beispiel illustrieren.

Die unterschiedliche Wirkung sehr ähnlicher Juvenilhormonmimics innerhalb einer Art, und die teilweise völlige Umkehrung der Aktivität der Vergleichsverbindungen für eine andere Insektenart sind für die Entwicklungsinhibitoren ausgeprägter, als für neurotoxische Substanzen. Aus der Literaturstelle 1 (oben) sowie aus G.B. Staal, 1982, INSECT CONTROL WITH GROWTH REGULATORS INTERFERING WITH THE ENDOCRINE SYSTEM, Ent. exp. & appl., 31, 15-23, wurden für die beiden unten angegebenen Wirkstoffe die Aktivitäten pro Gewichtseinheit errechnet.

| Substanz | Acyrtho-siphon pisum | Aedes aegypti | Heliothis viresc. | Galleria mellon | Tenebrio molit. | Musca dom. |
|---|---|---|---|---|---|---|
| 1 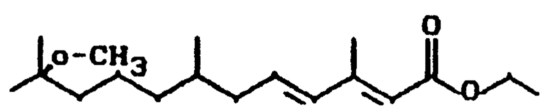 | 100 | 1 | 3 | 33 | 1 | 1 |
| 2 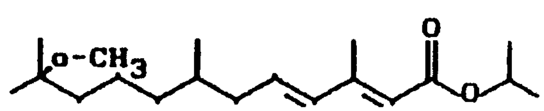 | 1 | 12 | 1 | 1 | 2255 | 1429 |

Dabei zeigt sich gute Wirkung von 1 bei Acyrthosiphon pisum und Galleria mellonella, bei denen 2 nur geringe Wirkung zeigt. Andererseits besitzt 2 gute Wirkung bei Tenebrio molitor und Musca domestica bei denen 1 nur geringe Wirkung zeigt.

Dieses Beispiel zeigt, wie schwierig gerade bei entwicklungshemmenden Verbindungen eine zuverlässige Vorhersage über die Wirkung ist.

Das heißt aber, selbst wenn aus EP-OS 128 648 bekannt war, daß die Verbindungen der Formel I insektizide Wirksamkeit besitzen, kann daraus nicht geschlossen werden, daß sie gerade gegen Flöhe (Siphonaptera) ausgezeichnet wirksam sind.

Zu den Flöhen zählen: Pulex irritans, Xenopsylla cheopis, Ctenocephalides canis, Ctenocephalides felis, Ceratophyllus gallinae, Echidnophaga gallinacea, Tunga penetrans.

Bevorzugt sind Verbindungen der Formel I in welcher

$R^1$ für Wasserstoff, Fluor, Chlor, Brom, $C_{1-4}$-Alkyl, $C_{1-4}$-Alkoxy, $C_{1-4}$-Alkylthio, $C_{1-4}$-Halogenalkyl mit bis zu 5-Halogenatomen, insbesondere Trichlormethyl, Trifluormethyl, $C_{1-4}$-Halogenalkoxy mit bis zu 5-Halogenatomen insbesondere Trichlormethoxy, Trifluormethoxy, $C_{1-4}$-Halogenalkylthio mit bis zu 4 Halogenatomen insbesondere Trifluormethylthio, CN, $NO_2$, $C_{2-3}$-Alkenyl, $C_{1-4}$-Alkoxy-$C_{1-2}$-alkyl, ins besondere Ethoxymethyl, Methoxyethyl, $C_{1-2}$-Alkoxy-$C_{1-4}$-alkoxy insbesondere Methoxyethoxy, $C_{1-4}$-Hydroxyalkoxy insbesondere Hydroxyethoxy, Dioxyethylen, Dioxymethylen, Difluordioxymethylen, Trifluordioxyethylen, Dichloroxymethylen steht,

$R^2$ für die bei $R^1$ angegebenen Reste steht,

$R^3$ für die bei $R^1$ angegebenen Reste steht,

$R^4$ für Wasserstoff, $C_{1-4}$-Alkyl $C_{1-4}$-Halogenalkyl mit bis zu 5 Halogenatomen, Chlor, Fluor, Brom steht,

$R^5$ für die bei $R^4$ angegebenen Reste steht,

Het für Pyridyl, Pyridazinyl, Pyrimidinyl, Pyrazinyl steht, die gegebenenfalls substituiert sind durch einen oder mehrere gleiche oder verschiedene Reste aus der Gruppe $C_{1-4}$-Alkyl, wie Methyl, Ethyl, t-Butyl, Halogen wie Fluor, Chlor, Brom, $NO_2$, $C_{1-4}$-Alkoxy, $C_{1-4}$-Alkylthio, $C_{1-4}$-Alkoxy, $C_{1-4}$-Alkylthio, $C_{1-4}$-Halogenalkyl mit bis zu 5 Halogenatomen, $C_{1-4}$-Halogenalkoxy mit bis zu 5 Halogenatomen, $C_{1-4}$-Halogenalkylthio mit bis zu 5-Halogenatomen,

X, Y unabhängig voneinander für -O-, -S- stehen

Z für -O-, -S-, $-CH_2-$, $-CHCH_3-$, $-C(CH_3)_2-$ steht,

m für 1 oder 2 steht,

n für 1 oder 2 steht.

Besonders bevorzugt sind Verbindungen der Formel I

in welcher

$R^1$ Wasserstoff, Methyl, Trifluormethyl, Methoxy, Trifluormethoxy, Chlor, Fluor steht,

$R^2$ für Wasserstoff steht,

$R^3$ für Wasserstoff, Fluor, Chlor, Methyl steht,

$R^4$ für Wasserstoff oder Methyl steht,

$R^5$ für Methyl, Ethyl, Trifluormethyl order Wasserstoff steht,

Het für Pyridyl oder Pyridazinyl steht die gegebenenfalls substituiert sind durch Fluor, Chlor, Methyl, $NO_2$, Methoxy, Methylmercapto,

X für 0 steht,

Y für 0 steht,

Z für O, $CH_2$, oder-$C(CH_3)_2$- steht,

m für 1 steht,

n für 1 steht.

Im einzelnen seien folgende Verbindungen genannt:

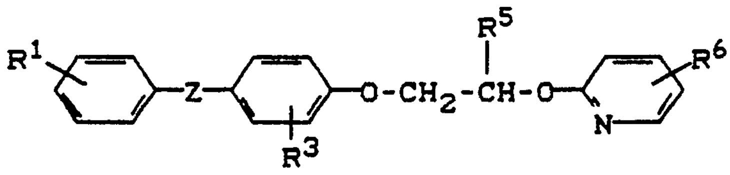

| $R^1$ | $R^3$ | $R^5$ | $R^6$ | Z |
|---|---|---|---|---|
| H | H | $CH_3$ | H | O |
| H | H | $CH_3$ | 2-Cl | O |
| 5-F | H | $CH_3$ | H | O |
| H | H | $CF_3$ | H | O |
| H | H | $C_2H_5$ | H | O |
| H | H | H | H | O |
| H | H | $CH_3$ | H | $CH_2$ |
| H | H | $CH_3$ | H | $C(CH_3)_2$ |

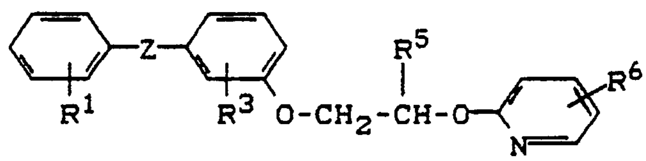

| $R^1$ | $R^3$ | $R^5$ | $R^6$ | Z |
|---|---|---|---|---|
| H | H | $CH_3$ | H | O |

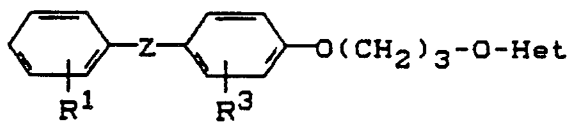

| $R^1$ | $R^3$ | Z | Het |
|---|---|---|---|
| H | H | O | 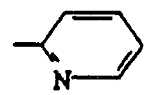 |
| H | H | O | 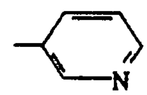 |
| H | H | O | 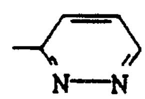 |

Die Anwendung der Wirkstoffe erfolgt direkt oder in Form von geeigneten Zubereitungen, dermal, enteral oder parenteral, oder durch Behandlung der Umgebung oder mit Hilfe wirkstoffhaltiger Formkörper wie z.B. Bänder, Halsbänder, Gliedmaßenbänder, Markierungsvorrichtungen.

Die dermale Anwendung geschieht z.B. in Form des Tauchens (Dippen), Sprühens (Sprayen) oder Aufgießens (pour-on und spot-on) und des Einpuderns.

Zubereitungen zur dermalen Anwendung sind z.B. Lösungen, Suspensions- und Emulsionskonzentrate sowie Mikroemulsionen, die vor Anwendung mit Wasser verdünnt werden, Aufgußformulierungen, Pulver und Stäube, Aerosole und wirkstoffhaltige Formkörper.

Lösungen, Suspensions- und Emulsionskonzentrate sowie Mikroemulsionen die vor der Anwendung mit Wasser verdünnt werden, Pulver, Stäube und Aerosolformulierungen werden zur Behandlung der Umgebung der Haustiere eingesetzt.

Diese Zubereitungen werden in bekannter Weise hergestellt, z.B. durch Vermischen des Wirkstoffs mit Streckmitteln, also z.B. flüssigen Lösungsmitteln, gegebenenfalls unter Verwendung von oberflächenaktiven Mitteln, also Emulgiermitteln und/oder Dispergiermitteln. Im Falle der Benutzung von Wasser als Streckmittel können z.B. auch organische Lösungsmittel als Hilfslösungsmittel verwendet werden.

Zu den flüssigen Verdünnungsmitteln zählen neben Wasser
Alkohole wie Methanol, Ethanol, Isopropanol, n-Butanol, Amylalkohol, Octanol;
Glykole wie Propylenglykol, 1,3-Butylenglykol, Ethylglykol, Dipropylenglykolmonomethylether;
Glycerin;
aromatische Alkohole wie Benzylalkohol;
Carbonsäureester wie z.B. Ethylacetat, Benzylbenzoat, Butylacetat, Propylencarbonat, Milchsäureethylester;
aliphatische Kohlenwasserstoffe wie Paraffine, Cyclohexan, Methylenchlorid, Ethylenchlorid,
aromatische Kohlenwasserstoffe wie Xylol, Toluol, Alkylnaphthaline, Chlorbenzole;
Ketone wie z.B. Aceton und Methylethylketon, Methylisobutylketon, Cyclohexanon;
natürliche und synthetische Mono- und Triglyceride mit natürlichen Fettsäuren wie Baumwollsaatöl, Erdnußöl, Maiskeimöl, Olivenöl, Ricinusöl, Sesamöl;
weiterhin Dimethylsulfoxid, Dimethylacetamid, Dimethylformamid, N-Methylpyrrolidon, Dioxan, 2,2-Dimethyl-4-oxymethyl-1,3-dioxolan.

Zu den oberflächenaktiven Stoffen zählen:
Emulgatoren und Netzmittel wie anionaktive Tenside, z.B. Alkylsulfonate, Alkylsulfate, Arylsulfonate, Na-Laurylsulfate, Fettalkoholethersulfate, Mono-Dialkylpolyglykoletherorthophosphorsäureester-Monoethanolaminsalz, Calciumalkylarylsulfonat;
kationaktive Tenside, z.B. Cetyltrimethylammoniumchlorid;
ampholytische Tenside, z.B. Di-Na-N-lauryl-betaiminodipropionat oder Lecithin;
nichtionogene Tenside, z.B. polyoxyethyliertes Ricinusöl, polyoxyethyliertes Sorbitan-Monooleat, polyoxyethyliertes Sorbitan-Monostearat, Glycerinmonostearat, Polyoxyethylenstearat, Alkylphenolpolyglykolether, polyoxyethyliertes Sorbitanmonopalmitat, Polyoxyethylenlaurylether, Polyoxyethylen-oleylether, Polyoxyethylenmannitanmonolaurat, Alkylpolyglykolether, Oleylpolyglykolether, Dodecylpolyglykolether, ethoxyliertes Nonylphenol, Isooctylphenolpolyethoxyethanol.

Die Zubereitungen können außerdem enthalten:

Haftvermittler, z.B. Carboxymethylcellulose, Methylcellulose und andere Cellulose- und Stärke-Derivate, Polyacrylate, Alginate, Gelatine, Gummi arabicum, Polyvinylpyrrolidon, Polyvinylalkohol, Copolymere aus Methylvinylether und Maleinsäureanhydrid, Polyethylenglykole, Paraffine, Öle, Wachse, hydriertes Rizinusöl, Lecithine und synthetische Phospholipide.

Die Zubereitungen können Farbstoffe wie anorganische Pigmente, z.B.Eisenoxid, Titanoxid, Ferrocyanblau und organische Farbstoffe, wie Alizarin-, Azo- und Metallphthalocyaninfarbstoffe enthalten.

Die Zubereitungen können Spreitmittel enthalten, z.B Silikonöle verschiedener Viskosität, Fettsäureester wie Ethylstearat, Di-n-butyl-adipat, Laurinsäurehexylester, Dipropylen-glykolpelargonat, Ester einer verzweigten Fettsäure mittlerer Kettenlänge mit gesättigten Fettalkoholen $C_{16}$-$C_{18}$, Isopropylmyristat, Isopropylpalmitat, Capryl/Caprinsäureester von gesättigten Fettalkoholen der Kettenlänge $C_{12}$-$C_{18}$, Isopropylstearat, Ölsäureoleylester, Ölsäuredecylester, Ethyloleat, Milchsäureethylester, wachsartige Fettsäureester, Dibutylphthalat, Adipinsäurediisopropylester, letzterem verwandte Estergemische u.a.;

Triglyceride wie Capryl/Caprinsäuretriglycerid, Triglyceridgemische mit Pflanzenfettsäuren der Kettenlänge $C_8$-$C_{12}$ oder anderen speziell ausgewählten natürlichen Fettsäuren, Partialglyceridgemische gesättigter oder ungesättigter evtl. auch hydroxylgruppenhaltige Fettsäuren, Monodiglyceride der $C_8$/$C_{10}$-Fettsäuren und andere;

Fettalkohole wie Isotridecylalkohol, 2-Octyldodecanol, Cetylstearylalkohol, Oleylalkohol.

Zur Herstellung von Pulvern und Stäuben wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Als solche dienen anorganische und organische Stoffe. Anorganische Stoffe sind gegebenenfalls gebrochene und fraktionierte, z.B. synthetische und natürliche Gesteinsmehle wie Kaoline, Talkum, Kreide, Diatomeenerde, Kochsalz, Aluminiumoxide, Kieselsäuren, Tonerden, gefälltes oder kolloidales Siliciumdioxid. Organische Stoffe sind Laktose, Stärke und Cellulose bzw. Cellulosederivate.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite.

Die Wirkstoffe können in Form ihrer oben erwähnten festen oder flüssigen Formulierungen auch eingekapselt vorliegen.

Die Wirkstoffe können auch in Form eines Aerosols angewendet werden. Dazu wird der Wirkstoff in geeigneter Formulierung unter Druck fein verteilt.

Es kann auch vorteilhaft sein, die Wirkstoffe in Formulierungen anzuwenden, die den Wirkstoff verzögert freigeben. Als solche seien wirkstoffhaltige Formkörper wie z.B. Platten, Bänder, Streifen, Halsbänder, Gliedmaßenbänder, Markierungsvorrichtungen genannt.

Die Wirkstoffe können in den Formulierungen allein oder in Mischung mit anderen Wirkstoffen oder Synergisten vorliegen.

Direkt angewendete Formulierungen enthalten zwischen $10^{-7}$ und 5 Gewichtsprozent, bevorzugt zwischen $10^{-4}$ und 1 Gewichtsprozent Wirkstoff.

Formlierungen die erst nach weiterer Verdünnung angewendet werden enthalten 1 - 95 Gewichtsprozent, bevorzugt 5 - 90 Gewichtsprozent Wirkstoff.

<u>Formulierungsbeispiele</u>

| 1. Herstellung eines Emulsionskonzentrates | | |
|---|---|---|
| a) | Wirkstoff gemäß Beispiel 1 (100 %)<br>nichtionischer Emulgator (Emulgator 368® = Alkylarylpolyglycolether MG ca. 1165)<br>Dipropylenglykolmonomethylether ad | 25,00 g<br>25,00 g<br>100,00 ml |

Herstellung

Die Substanzen werden zusammen gewogen und so lange gerührt, bis eine klare Lösung entstanden ist.
Vor Gebrauch wird die Lösung auf ihre Anwendungskonzentration verdünnt.

| | | |
|---|---|---|
| b) | Wirkstoff gemäß Beispiel 2 (100 %)<br>Polyoxyethylenstearat<br>Sorbitan-Sesquioleat<br>Wasser<br>Polyethylenglykol 200 ad 100 ml | 1,00 g<br>0,50 g<br>0,40 g<br>4,00 g |

Herstellung und Anwendung wie bei 1a

| 2. Herstellung einer pour-on-Formulierung | |
|---|---|
| a) Zusammensetzung | |
| Wirkstoff gemäß Beispiel 1 (100 %)<br>Isopropylmyristat<br>2-Octyldodecanol<br>Isopropanol ad 100 ml | 5,00 g<br>30,00 g<br>20,00 g |

Herstellung:

Die Substanzen werden zusammen gewogen und bis zur klaren Lösung gerührt.

| b) Zusammensetzung | |
|---|---|
| Wirkstoff gemäß Beispiel 1 (100 %)<br>Silikonöl 100<br>Butylacetat ad 100 ml | 0,50 g<br>30,00 g |

Herstellung wie bei Beispiel a)

| 3. Herstellung einer Mikroemulsion | |
|---|---|
| Wirkstoff gemäß Beispiel 1 (100 %)<br>Eumulgin B3®(Alkylarylpolyglycolether)<br>Cetiol HE® (Polyolfettsäureester)<br>Isopropylmyristat<br>Benzylalkohol<br>Wasser ad 100 ml | 13,00 g<br>30,00 g<br>30,00 g<br>5,00 g<br>3,00 g |

Herstellung:

Der Wirkstoff wird in den lipophilen Komponenten (Eumulgin, Cetiol, Benzylalkohol, Isopropylmyristat) dispergiert.

Nach Erwärmen auf 60° C wird Wasser der gleichen Temperatur zugemischt und abgekühlt. Die entstandene Mikroemulsion gleicht äußerlich einer klaren Lösung.

4. Herstellung einer Sprühformulierung

| Zusammensetzung | |
|---|---|
| Wirkstoff gemäß Beispiel 1 (100 %) | 20 g |
| Emulgator Toximul® (Mischung aus Alkylbenzolsulfonat-Ca und nichtionogenen Emulgatoren und Methanol) | 7 g |
| Emulgator Toximul S® (Mischung aus Alkylbenzolsulfonat-Ca und nichtionogenem Emulgator und Methanol) | 5 g |
| Sovesso 200® (Alkylnaphthalin-Gemisch hochsiedender Erdölfraktionen) | ad 100 ml |

Herstellung:

Der Wirkstoff wird mit den restlichen Komponenten zusammen gewogen, gerührt und vor Anwendung mit Wasser auf die Anwendungskonzentration verdünnt.

Herstellung der Wirkstoffe:

Beispiel 1

12,6 g 1-Methyl-2-(4-phenoxyphenoxy)-ethanol und 13 g 2-Chlorpyridin werden in 200 ml Toluol mit 12 g NaOH-Pulver, 8 g $K_2CO_3$-Pulver und 0,8 g des Phasentransfer Katalysators Tris-(3,6-dioxaheptyl)amin 24 h unter Rückfluß erhitzt. Nach dem Abkühlen wird die Toluolphase mit Wasser gewaschen über $Na_2SO_4$ getrocknet und im Vakuum eingeengt. Anschließend wird der Rückstand destilliert. Ausbeute: 9,5 g, Siedepunkt 250° C/0,4 mm.

Analog wurden die folgenden Verbindungen hergestellt:

Beispiel 2

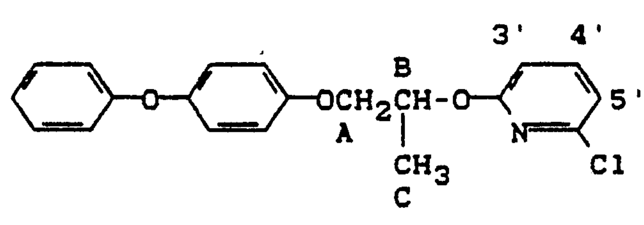

$^1$H-NMR, $CDCl_3$:

7,5-6,62 ppm (m, 12H, 9-Aromat. H + H-3′ + H-4′ + H-5′); 5,55 ppm (m, $CH_B$, 1H); 4.18-4.05 ppm (m, 2H, $CH_{2A}$); 1.48 ppm (d,3H, J = 7,5 Hz, $CH_{3C}$).

Siedepunkt:

250 °C/0,2 mm

Ausbeute:

33 % d.Th.

Beispiel 3

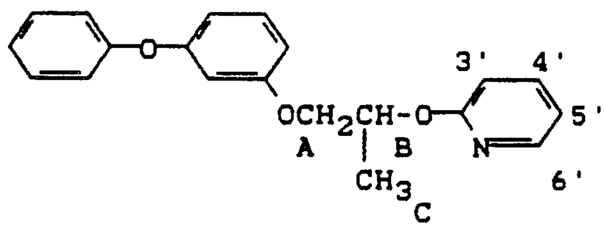

$^1$H-NMR, $CDCl_3$:

8,1 ppm (m, H-6′); 7,5 ppm (m, H-4′); 7,35 ppm-6,55 ppm (m, 11 H, 9-Aromat. H+H-3′+H-5′); 5,55 ppm (m, 1H, $CH_B$); 4,15 ppm-4.02 ppm (m, 2H, $CH_{2A}$); 1,45 ppm (d, J = 6,6 Hz; 3H, $CH_3C$).

Siedepunkt:

230 °C-250 °C/0,2 mm

Ausbeute:

25 % d.Th.

Beispiel 4

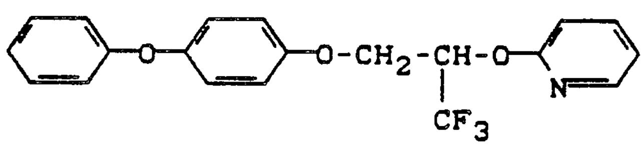

Siedepunkt:

250 °C/0,2 mm

Ausbeute:

16 % d.Th.

Beispiel 5

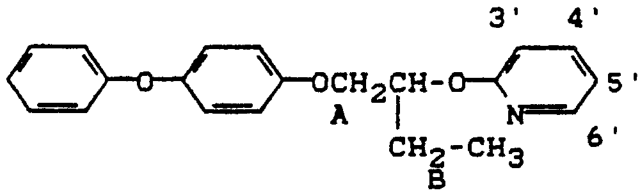

$^1$H-NMR, $CDCl_3$:

8,12 ppm (m, 1H, H-6′); 7,55 ppm (m, 1H, H-4′); 7.29-6.73 ppm (m, 11H, 9-Aromat. H + H-3′ + H-5′); 5,46 ppm (m, 1H, -CH-O-); 4,16 ppm (m, 2H, $CH_{2A}$); 1,93 ppm (m, 2H, $CH_{2B}$); 1.04 ppm (t, J = 6,5 Hz; 3H, $CH_3$).

Ausbeute:

40 % d.Th.

Beispiel 6

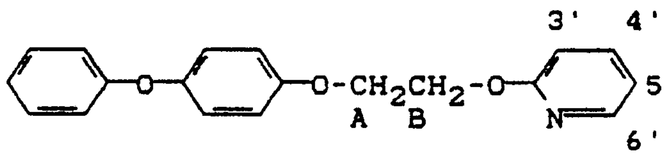

$^1$H-NMR, $CDCl_3$:

8,15 ppm (m, H-6′); 7,58 ppm (m, H-4′); 7,32-6.90 ppm (m, 9H, Aromat); 6.88 ppm (m, H-5′); 6.81 ppm (m, H-3′); 4.68 ppm (t, J = 4,9 Hz, $CH_{2B}$); 4.31 ppm (t, $CH_{2A}$).

Siedepunkt:

250° C/0,3 mm

Ausbeute:

66 % d.Th.

Beispiel 7

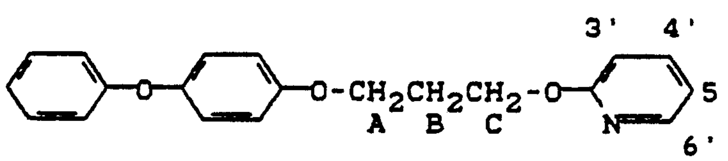

$^1$H-NMR, DMSO:

8,16-8,14 ppm (m, H-6′); 7,59-7,53 ppm (m, H-4′); 7.31-6.83 ppm (m, 10 H, 9-Aromat. H + H-5′); 6.73 ppm (m, H-3′); 4.49 ppm (t, J = 6,2 Hz; $CH_{2c}$); 4,13 ppm (t, $CH_{2A}$); 2,26 ppm (quint., $CH_{2B}$).

Ausbeute:

56 % d.Th.

Beispiel 8

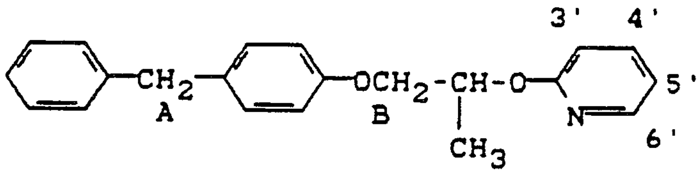

$^1$H-MNR, $CDCl_3$:

8,1 ppm (m, H-6'); 7,55 ppm (m, H-4'); 7.32-6.70 ppm (m, 11 H, 9-Aromat. H + H-3' + H-5'); 5,55 ppm (m, 1H, CH); 4.15-4.02 ppm (m, 2H, $CH_{2B}$); 3,88 ppm (s, $CH_{2A}$); 1,45 ppm (d, J = 7Hz, $CH_3$).

Ausbeute:

40 % d.Th.

Beispiel 9

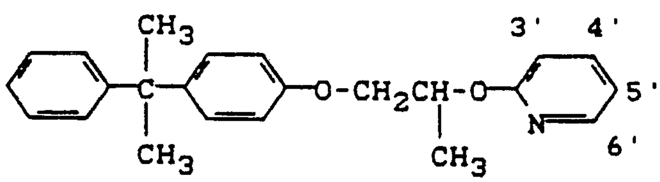

$^1$H-MNR, $CDCl_3$:

8,16 (m, H-6'); 7,69 ppm (m, H-4'); 7,28-6,77 ppm (m, 11H, 9-Aromat. H + H-3' + H-5'); 5,50 ppm (m, CH); 4,17-4,05 ppm (m, 2H, $CH_2$); 1,60 ppm (s, 6H,

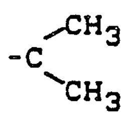 );

1,34 ppm (d, J = 6,4 Hz, $CH_3$).

Ausbeute:

45 % d. Th.

Anwendungsbeispiele

A. In vitro-Test an Flöhen (alle Entwicklungsstadien)

Testobjekt: Alle Stadien (Eier, Larven, Puppen, Adulte) von Ctenocephalides felis

Testverfahren

Die zu testende Substanz wird in der gewünschten Konzentration homogen in Blutmehl verteilt. In dieses Gemisch aus Blutmehl und Testsubstanz werden Floheier, die von flohbefallenen Katzen gesammelt wurden, gebracht und bei 80 % rel. Feuchte und 25 °C inkubiert.

Nach Ablauf der Entwicklungszeit von 3 - 4 Wochen wird festgestellt, ob adulte Flöhe zur Entwicklung gekommen sind. Dabei bedeutet eine 100 %ige Wirkung, daß keine adulten Flöhe festgestellt wurden.

Verbindung gemäß Beispiel 2 besitzt bei einer Konzentration von 5 ppm 100% Wirkung.

B. In vivo-Test an Flöhen (alle Entwicklungsstadien)

Testobjekt: Eier, Larven, Puppen von Ctenocephalides felis in der Umgebung von Hunden/Katzen, adulte Flöhe auf Hunden/Katzen.

Testverfahren

Läger von flohfreien Hunden/Katzen in Boxen werden mit der zu testenden Substanz in der gewünschten Anwendungskonzentration besprüht. Zu bestimmten Zeiten nach der Applikation werden Eier von Ctenocephalides felis, die von behandelten Katzen gesammelt wurden, auf die Läger der Hunde/Katzen gebracht. Es wird festgestellt, ob und zu welchem Zeitpunkt die in den Boxen befindlichen Hunde/Katzen von adulten Flöhen befallen werden.

Dabei bedeutet eine 100%ige Wirkung, daß Hunde/Katzen keinen Flohbefall aufweisen und 0% Wirkung, daß Hunde/Katzen von Flöhen befallen sind.

Verbindung gemäß Beispiel 2 besitzt 100% Wirkung.

**Ansprüche**

1. Verwendung von substituierten Alkoxydiphenylethern oder -diphenylmethanen der allgemeinen Formel (I)

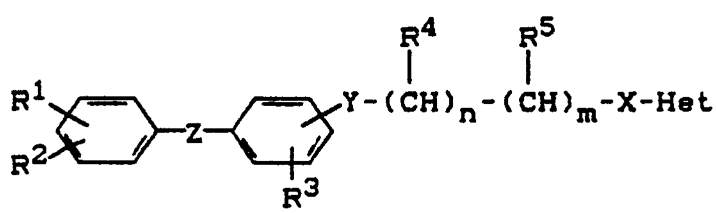

in welcher

$R^1$ für Wasserstoff, Halogen, Alkyl, Alkoxy, Alkylthio, Halogenalkyl, Halogenalkoxy, Halogenalkylthio, Dioxyalkylen, Dioxyhalogenalkylen, CN, $NO_2$, Alkenyl, Alkinyl, Alkoxyalkyl, Alkoxyalkoxy, Hydroxyalkoxy steht,

$R^2$ für die bei $R^1$ angegebenen Reste steht,

$R^3$ für die bei $R^1$ angegebenen Reste steht,

$R^4$ für Wasserstoff, Alkyl, Halogenalkyl oder Halogen steht,

$R^5$ für die bei $R^4$ angegebenen Reste steht,

Het für gegebenenfalls substituiertes Heteroaryl steht das nicht über das Heteroatom an den übrigen Rest gebunden ist,

X, Y unabhängig voneinander für -O-, oder -S-stehen

Z für -O-, -S-, $-CH_2-$, $-CHCH_3-$, $-C(CH_3)_2-$steht,

m und n unabhängig voneinander für 0, 1, 2, 3 stehen ihre Summe aber gleich oder größer 2 ist,

zur Bekämpfung von Flöhen.

2. Mittel zur Bekämpfung von Flöhen, gekennzeichnet durch einen Gehalt an mindestens einem substituierten Alkoxydiphenylether oder -diphenylmethan der Formel (I), gemäß Anspruch 1.

3. Verfahren zur Bekämpfung von Flöhen, dadurch gekennzeichnet, daß man substituierte Alkoxydiphenylether oder -diphenylmethane der Formel (I) gemäß Anspruch 1 auf Flöhe, ihre Wirtstiere und/oder ihren Lebensraum einwirken läßt.

4. Verfahren zur Herstellung von Mitteln zur Bekämpfung von Flöhen dadurch gekennzeichnet, daß man substituierte Alkoxydiphenylether oder -diphenylmethane der Formel (I) gemäß Anspruch 1 mit Streckmitteln und/oder oberflächenaktiven Mitteln vermischt.

5. Verwendung von substituierten Alkoxydiphenylethern oder -diphenylmethanen der Formel (I) gemäß Anspruch 1 zur Herstellung von Bekämpfungsmitteln gegen Flöhe.